# EUROPEAN PATENT SPECIFICATION

(11) **EP 2 672 843 B1**
(45) Date of publication and mention of the grant of the patent: **11.07.2018**
(21) Application number: 12704992.2
(22) Date of filing: 03.02.2012
(51) Int. Cl.: A23L 33/10, A23L 33/16

(54) **NUTRITIONAL PRODUCTS COMPRISING BETA-HYDROXY-BETA-METHYLBUTYRATE**
NAHRUNGSMITTEL MIT BETA-HYDROXY-BETA-METHYLBUTYRAT
PRODUITS À FINALITÉ NUTRITIONNELLE COMPORTANT DU BÊTA-HYDROXY-BÊTA-MÉTHYLBUTYRATE

(30) Priority: 07.02.2011 US 201161439950 P
(43) Date of publication of application: 18.12.2013
(73) Proprietor: ABBOTT LABORATORIES, Abbott Park, IL 60064-3500 (US)
(72) Inventor: JOHNS, Paul W., Columbus, Ohio 43221 (US); PEREIRA, Suzette L., Westerville, Ohio 43802 (US); KENSLER, Ann M., Sugar Grove, Ohio 43155 (US)
(74) Representative: Boult Wade Tennant
(86) International application number: PCT/US2012/023767
(87) International publication number: WO 2012/109105

(56) References cited:
- WO-A1-2012/088075
- WO-A1-2012/092035
- US-A1- 2005 215 640
- KREIDER R B ET AL: "EFFECT OF CALCIUM BETA-HYDROXY-BETA-METHYLBUTYRATE (HMB) SUPPLEMENTATION DURING RESISTANCE-TRAINING ON MARKERS OF CATABOLISM, BODY COMPOSITION AND STRENGTH", INTERNATIONAL JOURNAL OF SPORTS MEDICINE, THIEME, STUTTGART, DE, vol. 20, no. 8, 1 November 1999 (1999-11-01), pages 503-509, XP008037671, ISSN: 0172-4622, DOI: 10.1055/S-1999-8835
- DATABASE GNDP [Online] MINTEL; May 2010 (2010-05), ANONYMOUS: "RELOAD DIETARY SUPPLEMENTS", XP002676291, retrieved from www.gnpd.com Database accession no. 1315326
- JAMES E. CHARBONNEAU: "Recent case histories of food product -metal container interactions using scanning electron microscopy-x-ray microanalysis", SCANNING, vol. 19, no. 7, 1 October 1997 (1997-10-01), pages 512-518, XP55027103, ISSN: 0161-0457, DOI: 10.1002/sca.4950190710
- ANGELA MONTANARI ET AL: "Quality of organic coatings for food cans: evaluation techniques and prospects of improvement", PROGRESS IN ORGANIC COATINGS, vol. 29, no. 1-4, 1 September 1996 (1996-09-01), pages 159-165, XP55027106, ISSN: 0300-9440, DOI: 10.1016/S0300-9440(96)00625-X
- ANONYMOUS: "Lean DynamX", INTERNET CITATION, 1 January 2003 (2003-01-01), pages 1-5, XP002670342, Retrieved from the Internet: URL:http://www.fitpage.de/produkte/pd-1330 122620.htm?categoryId=181 [retrieved on 2012-02-24]
- Anonymous: "Preventing-oxidation-is-key-to-omega-3-fo rmulation", Internet , 19 August 2005 (2005-08-19), Retrieved from the Internet: URL:https://www.nutraingredients-usa.com/A rticle/2005/06/22/Preventing-oxidation-is- key-to-omega-3-formulation

## Description

### FIELD OF THE DISCLOSURE

The present disclosure relates to beta-hydroxy-beta-methylbutyrate- containing nutritional products that are oxidatively stable in the presence of iron.

### BACKGROUND OF THE DISCLOSURE

Nutritional products, comprising a targeted selection of nutrition ingredients generally including protein, lipids, vitamins and minerals are well known and widely available, some of which may provide a sole source of nutrition while others may provide a supplement source. These nutritional products include powders that can be reconstituted with water or other aqueous liquid, as well as ready to drink nutritional liquids such as milk or protein based emulsions or non-emulsified liquids. These nutritional products are often used to improve or maintain muscle health in athletes as well as individuals at risk of affliction with a disease or condition associated with the wasting of skeletal muscles.

A common problem in many protein-containing nutritional products is the metal-catalyzed oxidation of lipids (including DHA and ARA) and vitamins, as well as other oxidation-prone nutritional product components. Oxidation of these components may lead to measurable losses of the nutrients, including, for example, lipids, vitamin A, vitamin D, and folic acid, as well as increases in lipid oxidation off notes, generally occurring over the course of the product's shelf life. Iron and copper are generally regarded as the highly active species in this oxidative catalysis.

These nutrient oxidation problems have previously been addressed by fortifying nutritional products with conventional antioxidants such as vitamin E and ascorbyl palmitate; fortifying nutritional products with hydrolyzed proteins, which may exert antioxidant activity; using nitrogen blanketing to decrease headspace oxygen in the containers holding the nutritional products; and substituting partially soluble iron salts in the nutritional products to minimize iron-driven oxidation. To date, however, these solutions have not been able to completely inhibit the nutrient oxidation problems associated with many nutritional products.

It would therefore be desirable to formulate nutritional products with additional nutrients including vitamins and lipids such that the nutrients are oxidatively stable over solutions have not been able to completely inhibit the nutrient oxidation problems associated with many nutritional products.

Kreider et al, 1999, disclose supplementation of HMB to vitamin/mineral fortified carbohydrate powder. US2005/0215640 relates to complete nutritional products comprising HMB. WO2012/088075 (relevant under Art. 54(3) EPC only), relate to complete nutritional powders comprising calcium HMB and conjugated linolenic acid.

It would therefore be desirable to formulate nutritional products with additional nutrients including vitamins and lipids such that the nutrients are oxidatively stable over time, even in the presence of iron and copper. Additionally, it would be beneficial if the nutritional products had improved overall color and/or taste.

### SUMMARY OF THE DISCLOSURE

One embodiment of the disclosure not falling within the claims is directed to a nutritional powder comprising from about 0.1% to about 10% by weight beta-hydroxy-beta-methylbutyrate, from about 0.001% to about 5% by weight of iron, and at least one nutrient selected from fat, protein, or carbohydrate.

Another embodiment of the disclosure not falling within the claims is directed to a packaged composition comprising a metal container and a nutritional product packaged therein. The nutritional product comprises from about 0.1% to about 10% by weight beta-hydroxy-beta-methylbutyrate and at least one nutrient selected from fat, protein, or carbohydrate.

Another embodiment of the disclosure not falling within the claims is directed to a nutritional powder comprising from about 0.1% to about 10% by weight beta-hydroxy-beta-methylbutyrate, from about 0.001% to about 5% by weight of iron, and fat.
The present invention is directed to use of a nutritional powder to provide a reconstituted nutritional liquid by mixing the nutritional powder with water, the nutritional powder comprising from 0.1% to 10% by weight beta-hydroxy-beta- methylbutyrate, from 0.001% to 5% by weight iron, and from 5.0% to 10% by weight fat, wherein the fat comprises from 0.01% to 5% by weight of a long chain polyunsaturated fatty acid derived from fish oil, and wherein the weight ratio of beta-hydroxy- beta-methylbutyrate: iron is from 150:1 to 600:1.. The invention is as defined in the appended claims.

It has been found that the metal-catalyzed oxidation of nutrients such as lipids and vitamins in nutritional products can result in the products providing a reduced amount of nutrients to the end user. The products may also have a reduced shelf life. Additionally, in some cases the products may have unpleasing off notes and color.

It has been discovered that beta-hydroxy-beta-methylbutyrate (HMB) provides desirable antioxidant properties in nutritional products by forming an insoluble salt with undesirable soluble transition metal components, such as iron and copper, that are typically included in the nutritional product as nutrients, or that enter the product through contact with metal packaging. These insoluble salts have reduced oxidative reactivity as compared to their free soluble forms. As such, the inclusion of HMB in nutritional products may provide oxidative protection to the products by inhibiting and/or reducing the capacity of the oxidative components to catalyze the oxidation of oxidatively sensitive components, such as lipids and vitamins. Additionally, by inhibiting oxidation of nutrients, the inclusion of HMB in the nutritional products may prolong the shelf life, as well as improve flavor and color in the products.

### BRIEF DESCRIPTION OF THE FIGURES

Figures 1A and 1B depict the capacities of HMB and citric acid to bind to reactive iron over the concentration range of 10 mEq/L to 40 mEq/L as analyzed in Example 2.
Figures 2A and 2B depict the buffering capacity of HMB as analyzed in Example 3.
Figure 3 depicts the buffering capacity of HMB as analyzed in Example 4.
Figure 4 depicts the buffering capacity of HMB as analyzed in Example 4.
Figure 5 depicts the buffering capacity of HMB as analyzed in Example 4
Figure 6 depicts the buffering capacity of HMB as analyzed in Example 5.
Figure 7 depicts the buffering capacity of HMB as analyzed in Example 5.
Figure 8 depicts the buffering capacity of HMB as analyzed in Example 5.

### DETAILED DESCRIPTION OF THE DISCLOSURE

The packaged compositions and nutritional powders of the present disclosure comprise HMB to reduce or eliminate the oxidative activity of components such as iron, copper, zinc, and manganese in the compositions and powders. The essential features of the packaged compositions and nutritional powders and methods of making the packaged compositions and nutritional powders, as well as some of the many optional variations and additions, are described in detail hereafter.

The term "calcium HMB" as used herein, unless otherwise specified, refers to the calcium salt of beta-hydroxy-beta-methylbutyrate (also referred to as beta-dydroxyl-3-methyl butyric acid, beta-hydroxy isovaleric acid, or HMB), which is most typically in a monohydrate form. All weights, percentages, and concentrations as used herein to characterize calcium HMB are based on the weight of calcium HMB monohydrate, unless otherwise specified.

The term "nutritional powder" as used herein, unless otherwise specified, refers to nutritional powders that are generally flowable particulates and that are reconstitutable with an aqueous liquid, and which are suitable for oral administration to a human.

The terms "fat" and "oil" as used herein, unless otherwise specified, are used interchangeably to refer to lipid materials derived or processed from plants or animals. These terms also include synthetic lipid materials so long as such synthetic materials are suitable for oral administration to humans.

The term "shelf stable" as used herein, unless otherwise specified, refers to a nutritional product that remains commercially stable after being packaged and then stored at 18-24°C for at least 3 months, including from about 6 months to about 24 months, and also including from about 12 months to about 18 months.

The terms "nutritional product" as used herein, unless otherwise specified, refer to nutritional liquids and nutritional powders, the latter of which may be reconstituted to form a nutritional liquid, and are suitable for oral consumption by a human.

The term "nutritional liquid" as used herein, unless otherwise specified, refers to nutritional products in ready-to-drink liquid form and to nutritional liquids made by reconstituting the nutritional powders described herein prior to use.

The term "metal container" as used herein, unless otherwise specified, refers to a package within which the nutritional product is exposed to at least one metal surface, including packages where a minority of the interior surface area of the package is metal. The "metal container" includes at least one metal component selected from iron, copper, zinc and manganese.

All percentages, parts and ratios as used herein, are by weight of the total product, unless otherwise specified. All such weights as they pertain to listed ingredients are based on the active level and, therefore, do not include solvents or by-products that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the packaged compositions and nutritional powders of the present disclosure may also be substantially free of any optional or selected essential ingredient or feature described herein, provided that the remaining composition or powder still contains all of the required ingredients or features as described herein. In this context, and unless otherwise specified, the term "substantially free" means that the selected composition or powder contains less than a functional amount of the optional ingredient, typically less than about 1%, including less than about 0.5%, including less than about 0.1%, and also including zero percent, by weight of such optional or selected essential ingredient.

The packaged compositions and powders may comprise, consist of, or consist essentially of the essential elements of the products as described herein, as well as any additional or optional element described herein or otherwise useful in nutritional product applications.

### Product Form

Nutritional products may include nutritional liquids and nutritional solid forms, which include emulsified liquids, powders, including dry-mixed and/or spraydried powders that can be diluted with water or other aqueous liquids to form a nutritional liquid prior to use, nutritional bars and the like. The invention is the use of a powder that is diluted with water.

Nutritional products may be formulated with sufficient kinds and amounts of nutrients to provide a sole, primary, or supplemental source of nutrition, or to provide a specialized nutritional product for use in individuals afflicted with specific diseases or conditions or with a targeted nutritional benefit.

Additionally, in another embodiment, the product form includes a packaged composition that comprises a metal container and a nutritional product disposed therein. The nutritional product disposed within the metal container is a nutritional powder. Typically, the metal containers include at least one metal interior surface. In some embodiments, the metal container includes at least 10% metal on an interior surface, including from about 10% to about 90%, also including from about 15% to about 70%, and also including from about 20% to about 50%, and also including from about 25% to about 35%. The inclusion of HMB into the nutritional product disposed within the metal container is particularly beneficial as transition metals, including iron and copper, can migrate from the metal container into the nutritional product over time.

### Nutritional Solids

The solid forms of the nutritional products comprise HMB and fat and optionally protein, and/or carbohydrate and are in the form of a powder. These products are typically in the form of nutritional powders that are flowable or substantially flowable particulate compositions, or at least particulate compositions that can easily be scooped and measured with a spoon or similar other device, wherein the powder can easily be reconstituted by the intended user with water, in accordance with the invention, to form a nutritional formulation for immediate oral or enteral use. In this context, "immediate" use generally means within about 48 hours, typically within about 24 hours, most typically right after or within 20 minutes of reconstitution.

In one embodiment, the nutritional powders include HMB, at least a portion of which is HMB that has been spray dried with one or more of the other components of the nutritional powder. In some embodiments, 100% of the HMB present in the nutritional powders is HMB that has been spray dried with one or more of the other components of the nutritional powder. Alternatively, the nutritional powder may contain drymixed or dryblended HMB in an amount up to 100% of the total concentration of HMB.

### Nutritional Liquids

Nutritional liquids, including nutritional emulsions, comprise HMB and may be aqueous emulsions further comprising protein, fat and carbohydrate. These liquids are flowable or drinkable liquids at from about 1 to about 25°C and are typically in the form of oil-in-water, water-in-oil, or complex aqueous emulsions, although such emulsions are most typically in the form of oil-in-water emulsions having a continuous aqueous phase and a discontinuous oil phase.

Nutritional liquids may be and typically are shelf stable. The nutritional liquids typically contain up to about 95% by weight of water, including from about 50% to about 95%, also including from about 60% to about 90%, and also including from about 70% to about 85%, of water by weight of the nutritional liquid.

Nutritional liquids may have a variety of product densities, but most typically have a density greater than about 1.01 g/ml, including from about 1.06 g/ml to about 1.12 g/ml, and also including from about 1.085 g/ml to about 1.10 g/ml.

Nutritional liquids may have a caloric density tailored to the nutritional needs of the ultimate user, although in most instances the liquids comprise from about 100 to about 500 kcal/240 ml, including from about 150 to about 350 kcal/240 ml, and also including from about 200 to about 320 kcal/240 ml.

Nutritional liquid may have a pH ranging from about 2.5 to about 8, but are most advantageously in a range of from about 4.5 to about 7.5, including from about 5.5 to about 7.3, and including from about 6.2 to about 7.2.

### Beta-Hydroxy-Beta-Methylbutyrate (HMB)

The nutritional products comprise HMB, which means that the products are either formulated with the addition of HMB, most typically as a calcium monohydrate, or are otherwise prepared so as to contain HMB in the finished product. Any source of HMB is suitable for use herein provided that the finished product contains HMB, although such a source is preferably calcium HMB and is most typically added as such to the nutritional products during formulation.

Although calcium HMB monohydrate is the preferred source of HMB for use herein, other suitable sources may include HMB as the free acid, a salt, an anhydrous salt, an ester, a lactone, or other product forms that otherwise provide a bioavailable form of HMB from the nutritional product. Non- limiting examples of suitable salts of HMB for use herein include HMB salts, hydrated or anhydrous, of sodium, potassium, magnesium, calcium, or other non-toxic salt form. Calcium HMB monohydrate is preferred and is commercially available from Technical Sourcing International (TSI) of Salt Lake City, Utah and from Lonza Group Ltd. (Basel, Switzerland).

The concentration of HMB in the nutritional liquids may range up to about 10%, including from about 0.01% to about 10%, and also including from about 0.1% to about 5.0%, and also including from about 0.3% to about 2%, and also including from about 0.4% to about 1.5%, and also including from about 0.3% to about 0.6% by weight of the nutritional liquid. In one specific embodiment, the HMB is present in the nutritional liquid in an amount of about 0.67%, by weight of the nutritional liquid.

The total concentration of calcium HMB in the nutritional powders may range up to about 10%, including from about 0.1 % to about 8%, and also including from about 0.2% to about 5.0%, and also including from about 0.3% to about 3%, and also including from about 0.3% to about 1.5%, and also including from about 0.3% to about 0.6% by weight of the nutritional powder. All or a portion of the HMB in the nutritional powder may be spray dried HMB, as discussed herein.

The total concentration of HMB in the nutritional powder is described in relation to the amount of iron. Specifically, the weight ratio of HMB to iron is from 150:1 to 600:1 , desirably from 180:1 to 600:1 , and desirably from 200:1 to 600:1.

The nutritional products may provide from about 0.1 to about 10 grams/day of HMB. Accordingly, the nutritional products may provide from about 0.5 to about 2.5 grams, including from about 1.0 to about 1.7 grams, including about 1.5 grams of HMB per serving, wherein a serving may be about 240 ml of ready to feed nutritional liquid or about 240 ml of reconstituted nutritional solid. In one specific embodiment, HMB is provided at a level of about 1.58 grams per 240 ml. An individual may be administered one serving per day, two servings per day, three servings per day, or four or more servings per day to receive the desired amount of HMB from the nutritional product.

### Oxidatively Active Component

The nutritional products of some embodiments of the present disclosure may include oxidatively active components; that is, components that actively catalyze or otherwise result in the oxidation of at least one nutrient in the nutritional product. For example, many nutritional products may include a transition metal component, such as iron, zinc, manganese, or copper, that may modify the physical, nutritional, chemical, hedonic or processing characteristics of the products, or serve as pharmaceutical or additional nutritional components when used in a targeted population. Generally, the transition metal component is present in the nutritional product in a concentration of from about 0.00001% to about 5%, including from about 0.0001% to about 1%, including from about 0.00001% to about 0.1% by weight of the nutritional product.

In some nutritional products, however, these metal components may cause unwanted oxidation of sensitive nutrient such as vitamins (including, for example, vitamin C, vitamin A, vitamin D, vitamin B12, vitamin E, and folic acid), lipids (including, for example, fish oil, marine oil, DHA and ARA), and proteins.

In some embodiments, the nutritional product is packaged in a metal container, or a container that is partially made of metal, such that the product is in at least partial contact with the metal. Many metal containers may include metal components such as iron, copper, zinc and manganese that may transition or migrate into the nutritional product packaged therein. Over time, the concentration of these metal components can increase to levels sufficient to catalyze the oxidation reactions that result in unwanted oxidation of desirable nutrients.

### Macronutrients

Nutritional products may further comprise one or more optional macronutrients including proteins, lipids, carbohydrates, and combinations thereof. The nutritional products are desirably formulated to contain all three macronutrients. The reconstituted liquids of the present invention comprise fat.

Macronutrients suitable for use herein include any protein, lipid, or carbohydrate or source thereof that is known for or otherwise suitable for use in an oral nutritional liquid, provided that the optional macronutrient is safe and effective for oral administration and is otherwise compatible with the other ingredients in the nutritional product.

The concentration or amount of optional lipid, carbohydrate, and protein in the nutritional liquid can vary considerably depending upon the particular nutritional application of the product. These optional macronutrients are most typically formulated within any of the embodied ranges described in the following tables.

| Nutrient (% total calories) | Example A | Example B | Example C |
|---|---|---|---|
| Carbohydrate | 0-100 | 10-70 | 40-50 |
| Lipid | 0-100 | 20-65 | 35-55 |
| Protein | 0-100 | 5-40 | 15-25 |

Each numerical value preceded by the term "about"

| Nutrient (wt% composition) | Example D | Example E | Example F |
|---|---|---|---|
| Carbohydrate | 0-98 | 1-50 | 10-30 |
| Lipid | 0-98 | 1-30 | 3-15 |
| Protein | 0-98 | 1-30 | 2-10 |

Each numerical value preceded by the term "about"

### Protein

Any protein or source thereof that is suitable for use in oral nutritional products and is compatible with the essential elements and features of such products is suitable.

The concentration of protein in the nutritional liquid may range from about 1.0% to about 30%, including from about 1% to about 15%, and also including from about 1% to about 10%, and also including from about 1% to about 7%, by weight of the nutritional liquid.

For powder embodiments, the concentration of protein may range from about 1.0% to about 50%, including from about 10% to about 50%, and also including from about 10% to about 30%, by weight of the nutritional powder.

Non-limiting examples of suitable protein or sources thereof for use in the nutritional products include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy or pea) or combinations thereof. Non- limiting examples of such proteins include milk protein isolates, milk protein concentrates, casein protein isolates, whey protein, sodium or calcium casemates, whole cow's milk, partially or completely defatted milk, soy protein isolates, soy protein concentrates, and so forth.

### Carbohydrate

Any carbohydrate that is suitable for use in an oral nutritional product and is compatible with the essential elements and features of such products is suitable.

Carbohydrate concentrations in the nutritional liquid, for example, may range from about 5% to about 40%, including from about 7% to about 30%, including from about 10% to about 25%, by weight of the nutritional liquid. In one specific embodiment, the carbohydrate is present in the nutritional liquid in an amount of about 10.2%, by weight of the nutritional liquid.

Carbohydrate concentrations in the nutritional powder may range from about 10% to about 90%, including from about 20% to about 80%, further including from about 40% to about 60%, by weight of the nutritional powder. In one specific embodiment, the carbohydrate is present in the nutritional powder in an amount of about 58%, by weight of the nutritional powder.

Non-limiting examples of suitable carbohydrates or sources thereof for use in the nutritional products described herein may include maltodextrin, hydrolyzed or modified starch or cornstarch, glucose polymers, corn syrup, corn syrup solids, rice-derived carbohydrates, sucrose, glucose, fructose, lactose, high fructose corn syrup, honey, sugar alcohols (e.g., maltitol, erythritol, sorbitol), artificial sweeteners (e.g., sucralose, acesulfame potassium, stevia) and combinations thereof. A particularly desirable carbohydrate is a low dextrose equivalent (DE) maltodextrin.

### Fat

Any fat, most typically as emulsified fat that is suitable for use in an oral nutritional product and is compatible with the essential elements and features of such products is suitable.

The fat may be present in the nutritional liquids in an amount of from about 1% to about 30%, including from about 1% to about 20%, including from about 1% to about 15%, and also including from about 1.5% to about 5%, by weight of the nutritional liquid. In one specific embodiment, the nutritional liquid includes fat in an amount of about 1.6%, by weight of the nutritional liquid.

The fat is present in the nutritional powder from 5.0% to 10%, by weight of the nutritional powder. In one specific embodiment, the nutritional powder includes fat in an amount of about 7.5%, by weight of the nutritional powder.

Non-limiting examples of suitable fats or sources thereof for use in the nutritional products described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, fish oils, cottonseed oils, and combinations thereof.

The fat includes a long chain polyunsaturated fatty acid (LC-PUFA). LC-PUFAs, and particularly omega-3 and omega- 6 PUFAs. They may provide nutritional benefits to the user, such as helping to maintain the performance of the heart and cardiovascular system, reducing the levels of triglycerides (fats) in the blood, helping to regulate blood pressure and helping to maintain a regular heart beat. In addition, omega-3 LC-PUFAs have been shown to be helpful in maintaining healthy bones and joints and a healthy brain. These LC-PUFAs may generally be oxidatively unstable over extended time when included in nutritional products that also include metals, such as iron and copper. As noted above, by including HMB in the nutritional products, the oxidation rate over time of LC-PUFAs may be reduced.

Exemplary LC-PUFAs for use in the nutritional products include arachidonic acid (ARA), docosahexaenoic acid (DHA), eicosapentaenoic acid (EPA), linoleic acid, linolenic acid (alpha linolenic acid) and gamma-linolenic acid derived from oil sources such as plant oils, marine plankton, fungal oils, and fish oils. The LC-PUFAs of the invention are derived from fish oils such as menhaden, salmon, anchovy, cod, halibut, tuna, or herring oil.

These LC-PUFAs are included in an amount of from 0.01% to 5% by weight total fat in the powder.

### Optional Ingredients

The nutritional products comprising HMB may further comprise other optional ingredients that may modify the physical, nutritional, chemical, hedonic or processing characteristics of the products or serve as pharmaceutical or additional nutritional components when used in a targeted population. Many such optional ingredients are known or otherwise suitable for use in other nutritional products and may also be used in the nutritional products described herein, provided that such optional ingredients are safe and effective for oral administration and are compatible with the essential and other ingredients in the selected product form.

Non-limiting examples of such optional ingredients include preservatives, antioxidants, emulsifying agents, buffers, fructooligosaccharides, chromium picolinate, pharmaceutical actives, additional nutrients as described herein, colorants, flavors, thickening agents and stabilizers, and so forth.

The products may further comprise vitamins or related nutrients, non-limiting examples of which include vitamin A, vitamin D, vitamin E, vitamin K, thiamine, riboflavin, pyridoxine, vitamin B12, carotenoids, niacin, folic acid, pantothenic acid, biotin, vitamin C, choline, inositol, salts, and derivatives thereof, and combinations thereof. Particularly preferred embodiments include one or more of the following vitamins: folic acid, vitamin B12, vitamin C, vitamin D, and vitamin E.

When used in the nutritional products, the amount of vitamins will vary depending on the specific vitamin used, as well as the other components of the nutritional products. For example, when the nutritional product includes folic acid, the nutritional product typically includes from about 0.000001% to about 0.001% by weight of folic acid, including from about 0.000005% to about 0.0002% by weight, and including from about 0.00001% to about 0.0001% by weight of the nutritional product.

When vitamin D is included in the nutritional product, vitamin D is included in the nutritional product in amounts of from about 0.0000001% to about 0.00001%, including from about 0.0000005% to about 0.000002%, and including from about 0.0000003% to about 0.000003% by weight of the nutritional product.

The products may further comprise additional minerals, non-limiting examples of which include phosphorus, magnesium, calcium, sodium, potassium, molybdenum, chromium, selenium, chloride, and combinations thereof.

The products may also include one or more flavoring or masking agents. Suitable flavoring or masking agents include natural and artificial sweeteners, sodium sources such as sodium chloride, and hydrocolloids, such as guar gum, xanthan gum, carrageenan, gellan gum, gum acacia and combinations thereof.

### Methods of Manufacture

The nutritional liquids may be manufactured by any known or otherwise suitable method for making nutritional liquids, including emulsions such as milk-based nutritional emulsions.

In one suitable manufacturing process, a nutritional liquid is prepared using at least three separate slurries, including a protein-in-fat (PIF) slurry, a carbohydrate-mineral (CHO-MIN) slurry, and a protein-in-water (PIW) slurry. The PIF slurry is formed by heating and mixing the selected oils (e.g., canola oil, corn oil, fish oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. Avicel, gellan, carrageenan), and HMB. The resulting CHO-MIN slurry is held for 10 minutes with continued heat and agitation before adding additional minerals (e.g., potassium chloride, magnesium carbonate, potassium iodide, etc.) and/or carbohydrates (e.g., fructooligosaccharide, sucrose, corn syrup, etc.). The PIW slurry is then formed by mixing with heat and agitation the remaining protein (e.g., sodium caseinate, soy protein concentrate, etc.) into water.

The resulting slurries are then blended together with heated agitation and the pH adjusted to the desired range, typically from 6.6-7.0, after which the composition is subjected to high-temperature short-time (HTST) processing during which the composition is heat treated, emulsified and homogenized, and then allowed to cool. Water soluble vitamins and ascorbic acid are added, the pH is again adjusted to the desired range if necessary, flavors are added, and water is added to achieve the desired total solid level. The composition is then aseptically packaged to form an aseptically packaged nutritional emulsion, or the composition is added to retort stable containers and then subjected to retort sterilization to form retort sterilized nutritional emulsions.

The manufacturing processes for the nutritional emulsions may be carried out in ways other than those set forth herein without departing from the spirit and scope of the present disclosure. The present embodiments are, therefore, to be considered in all respects illustrative and not restrictive and that all changes and equivalents also come within the description of the present disclosure.

The nutritional solid, such as a spray dried nutritional powder or dry-mixed nutritional powder, may be prepared by any collection of known or otherwise effective techniques, suitable for making and formulating a nutritional powder.

For example, when the nutritional powder is a spray-dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid comprising HMB, and optionally protein, carbohydrate, and fat, and then spray drying the slurry or liquid to produce a spray dried nutritional powder. The method may further comprise the step of spray drying, dry mixing, or otherwise adding additional nutritional ingredients, including any one or more of the ingredients described herein, to the spray dried nutritional powder.

The methods of manufacture are preferably formulated with calcium HMB, which is most typically formulated as calcium HMB monohydrate, as the HMB source for use in the methods.

### Methods of Use

The nutritional products may be administered orally as needed to provide the desired level of nutrition, most typically in the form of one to two servings daily, in one or two or more divided doses daily, e.g., serving sizes typically ranging from about 100 to about 300 mL, including from about 150 to about 250 mL, including from about 190 mL to about 240 mL.

The use of HMB in the nutritional products provides an oxidatively shelf stable product having reduced off-color that is useful as a nutrition source. Particularly, when used in a nutritional product with an oxidatively active component such as iron or copper for example, HMB reduces the ability of the oxidatively active component to catalyze oxidation of sensitive nutrients in the product. For example, when HMB is used with the oxidatively active component iron, HMB forms an insoluble ferric salt with the soluble iron in the product. The insoluble salt form has significantly lower oxidative activity as compared to that of the soluble iron. This reaction inhibits the capacity of iron to catalyze the oxidation of oxidizable components such as lipids, vitamins, and protein side chains, including methionine.

### EXAMPLES

The following examples illustrate specific embodiments and or features of the nutritional products of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the product, unless otherwise specified.

The exemplified products are nutritional products prepared in accordance with manufacturing methods well known in the nutrition industry for preparing nutritional emulsions and powders.

### Example 1

In this Example, the capacity of HMB for binding iron is compared to that of various organic and mineral acids.

Prior to preparing the sample solutions, HMB free acid is prepared by cation exchange removal of the calcium from CaHMB (Lonza Group Ltd., Basel, Switzerland). All sample solutions are prepared by mixing the selected acid in water at a concentration of 26.15 mM. The pH of the sample solutions is adjusted to 7.0 with sodium hydroxide (NaOH). Iron is added at 20 mg/L as ferrous fumarate, and the solutions are stirred for three hours at room temperature (RT).

The samples are then prepared for "reactive iron" determination by centrifuging at 10,000 X g for five minutes and then diluting the supernatant (2 volumes to 10 volumes) in 0.10 M sodium acetate at a pH of 4.0. The analysis is conducted using ferrozine colorimetry. "Reactive iron" is the iron that complexes with the ferrozine reagent to yield the color measured by spectrometry. The results are shown in Table 1.

**Table 1**

| **Acid (at 26.15 mM, pH adjusted to 7.0)** | **Iron added as ferrous fumarate, mg/L** | **Reactive iron after 3h/RT, mg/L** | **Reactive iron, % of added iron** |
|---|---|---|---|
| Control (No acid) | 20.0 | 13.2 | 66% |
| Hydrochloric Acid | 20.0 | 14.4 | 72% |
| Citric Acid | 20.0 | 11.7 | 58% |
| 2,2-HMB^{a} | 20.0 | 5.30 | 26% |
| 2,3-HMB^{b} | 20.0 | 4.20 | 21% |
| HMB^{c} | 20.0 | 3.61 | 18% |
| Phosphoric Acid | 20.0 | 1.23 | 6% |
| Acetic Acid | 20.0 | 0.63 | 3% |

| | | | |
|---|---|---|---|
| ^{a}2-hydroxy-2-methylbutyric acid ^{b}2-hydroxy-3-methylbutyric acid ^{c}3-hydroxy-3-methylbutyric acid | | | |

As shown in Table 1, the only acid that increased reactive iron versus the acid-free control is the strong mineral acid hydrochloric acid. All of the organic acids, including citric acid, decreased reactive iron, due to iron salt formation at the neutral pH. Notably, HMB decreased reactive iron also, which is indicative of HMB's ability to bind iron and reduce its reactivity, and hence its oxidative catalytic ability.

### Example 2

In this Example, the capacity of HMB, over the concentration range of 10 mEq/L (1.2 g/L) to 40 mEq/L (4.7 g/L), to bind iron is compared to that of equivalent concentrations of citric acid.

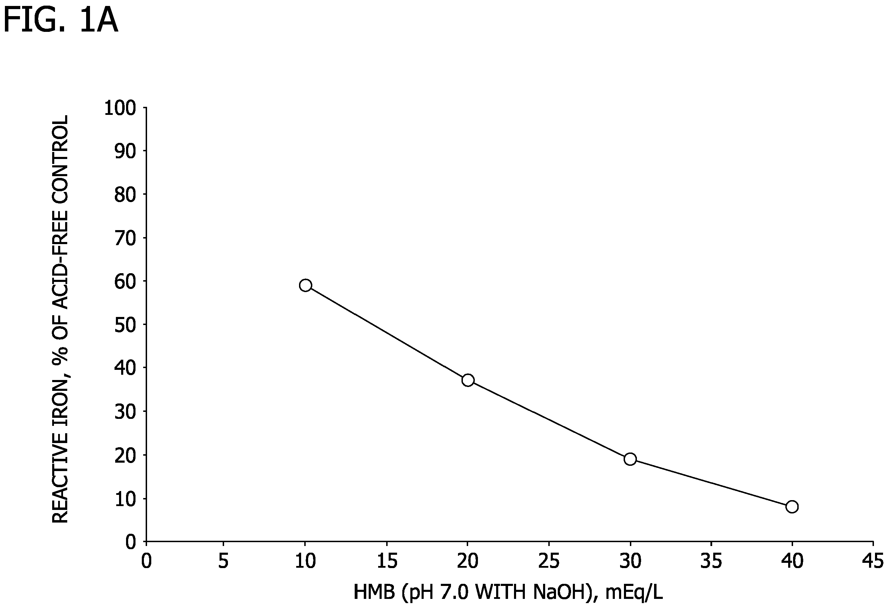

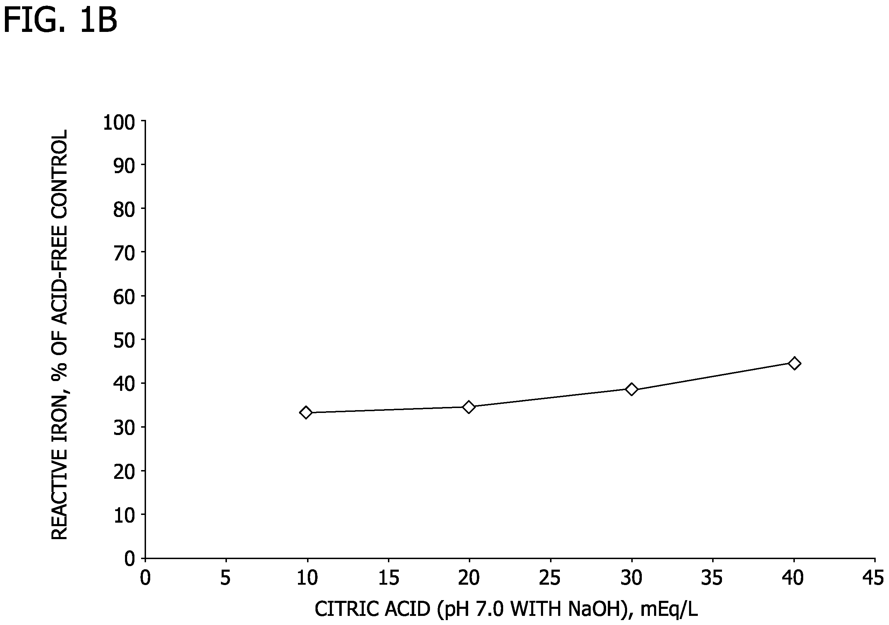

Sample solutions are prepared and analyzed as described in Example 1. The results are shown in Table 2, as well as Figures 1A and 1B.

**Table 2**

| **Acid concentration, mEq/L** | **Citric Acid, pH 7 w/ NaOH** | | **HMB, pH 7 w/ NaOH** | |
|---|---|---|---|---|
| | Reactive iron, mg/L | Reactive iron, % of control | Reactive iron, mg/L | Reactive iron, % of control |
| 10 | 5.6 | 33% | 9.9 | 59% |
| 20 | 5.8 | 34% | 6.2 | 37% |
| 30 | 6.4 | 38% | 3.2 | 19% |
| 40 | 7.4 | 44% | 1.4 | 8.3% |

As shown in Table 2 and Figures 1A and 1B, reactive iron increases as the concentration of citric acid increases, but decreases as the concentration of HMB increases. This result appears to be attributable to the formation of soluble iron complexes by citric acid, in contrast to the formation of insoluble ferric salts by HMB. Accordingly, the addition of HMB is shown to reduce the amount of reactive iron in solution, such that the iron would not be available for catalytic oxidation.

### Example 3

In this Example, the buffering capacity of adding HMB to the commercially available Ensure® Plus nutritional emulsion is analyzed.

HMB is obtained from Lonza as described in Example 1. The Ensure® Plus emulsions (available from Abbott Nutrition, Columbus, Ohio) used herein are described in Table 3.

**Table 3**

| | **Ensure Plus® with HMB** | **Ensure Plus® Control** |
|---|---|---|
| Milk Protein Caseinate | 70% (by weight protein) | 70% (by weight protein) |
| Soy Protein Concentrate | 25% (by weight protein) | 25% (by weight protein) |
| Whey Protein Concentrate | 5% (by weight protein) | 5% (by weight protein) |
| Citrate | 2160 mg/kg of emulsion | 2453 mg/kg of emulsion |
| Phosphate | 2380 mg/kg of emulsion | 2400 mg/kg (total phosphate) of emulsion |
| Mg(OH)₂ | 900 mg/kg emulsion | N/A |
| CaHMB | 6000 mg/kg of emulsion | N/A |

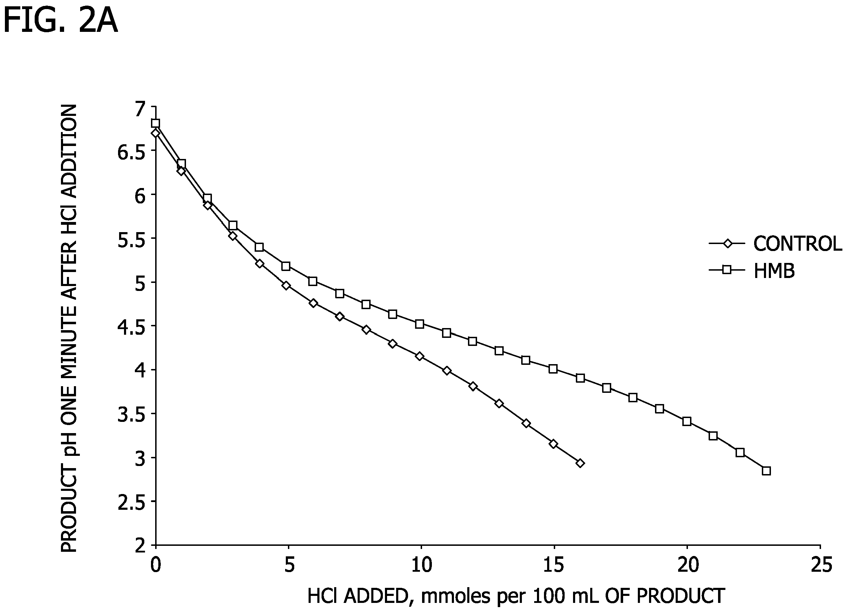

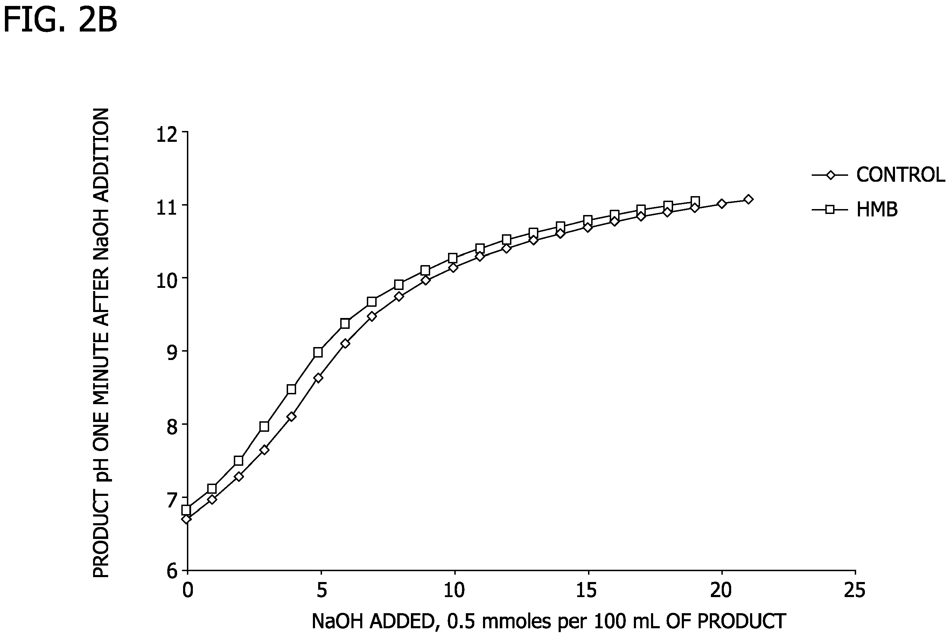

The buffering capacity of the sample emulsions is compared via HCl titration and via NaOH titration. Specifically, the buffering capacity is analyzed by determining the millimoles of HCl to lower the pH of 100 mL of nutritional emulsion from a pH of 6.0 to a pH of 3.0. Similarly, the millimoles of NaOH required to raise the pH of the 100 mL nutritional emulsion from a pH of 7.0 to a pH of 11.0 is also determined. The results are shown in Table 4, as well as in Figures 2A and 2B.

**Table 4**

| | **Ensure Plus® with HMB** | **Ensure Plus® Control** |
|---|---|---|
| HCl required to lower pH of 100 mL of emulsion from 6.0 to 3.0 mmoles | 21.0 | 13.9 |
| NaOH required to raise pH of 100 mL of emulsion from 7.0 to 11.0 mmoles | 9.04 | 9.62 |

As shown in Table 4 and Figures 2A and 2B, the Ensure Plus emulsion including HMB is significantly more resistant to pH lowering than the control emulsion. A resistance to pH change, such as is provided by the addition of HMB to the nutritional product, may decrease the release of bound minerals (e.g., phosphate-bound calcium), which, in ionic form (i.e., if released from the phosphate salt), may compromise the physical stability of the product and increase catalytic oxidation of oxidatively active components of the reconstituted products, such as iron and copper.

### Example 4

In this Example, the buffering capacity of adding HMB to the commercially available PediaSure® nutritional powder that has been reconstituted is analyzed.

HMB is obtained from Lonza as described in Example 1. Two samples of reconstituted nutritional powder are prepared. The first sample is a control sample of reconstituted PediaSure® nutritional powder (available from Abbott Nutrition, Columbus, Ohio). The second sample is reconstituted PediaSure® nutritional powder that has been fortified with 5.17 g/kg powder HMB. Prior to the addition of free HMB to the nutritional powder, the pH of the powder is adjusted to 6.7 with NaOH.

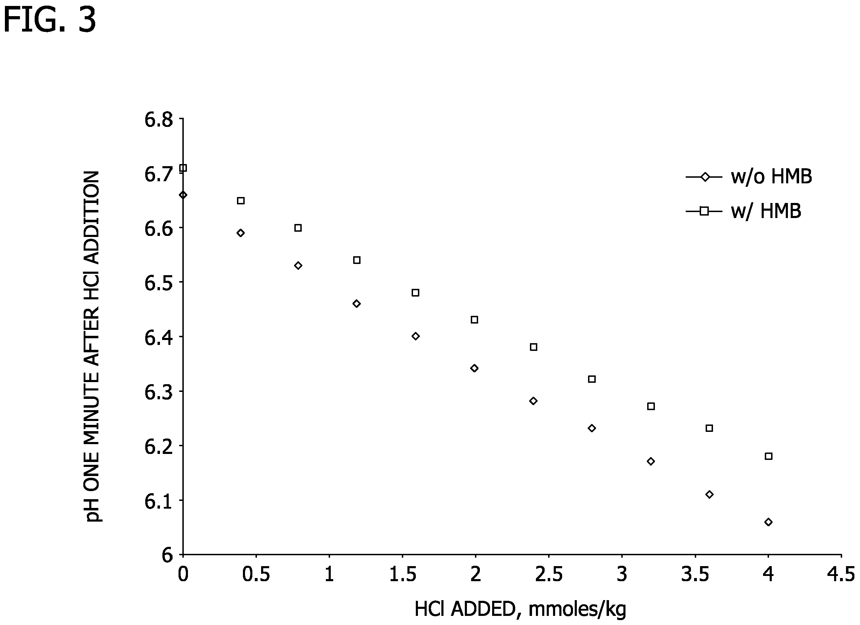

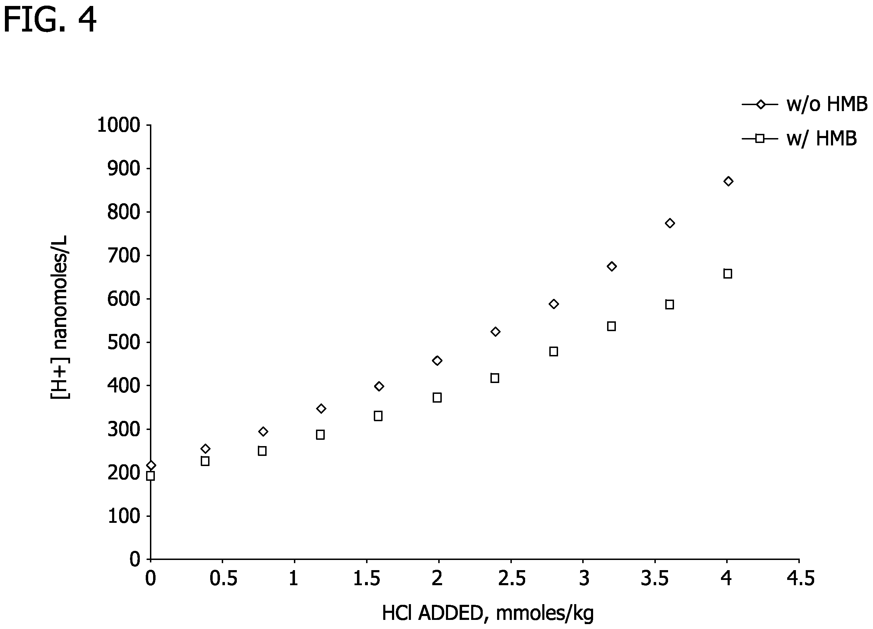

The buffering capacity of the samples is compared via HCl titration. Specifically, HCl is slowly added to the samples and the pH is measured one minute after each HCl addition. The hydrogen ion concentration ([H+]) is calculated from the pH values, and the results are shown in Table 5, as well as in Figures 3 and 4.

**Table 5**

| **HCl added, mmoles/kg** | **PediaSure® Powder w/o HMB addition** | | **PediaSure® Powder with HMB addition** | |
|---|---|---|---|---|
| | pH | [H+], nmoles/kg | pH | [H+], nmoles/kg |
| 0 | 6.66 | 218 | 6.71 | 194 |
| 0.40 | 6.59 | 256 | 6.65 | 228 |
| 0.80 | 6.53 | 294 | 6.60 | 251 |
| 1.20 | 6.46 | 346 | 6.54 | 288 |
| 1.60 | 6.40 | 397 | 6.48 | 330 |
| 2.00 | 6.34 | 456 | 6.43 | 371 |
| 2.40 | 6.28 | 523 | 6.38 | 416 |
| 2.80 | 6.23 | 587 | 6.32 | 477 |
| 3.20 | 6.17 | 674 | 6.27 | 536 |
| 3.60 | 6.11 | 774 | 6.23 | 587 |
| 4.00 | 6.06 | 869 | 6.18 | 659 |
| **Change** | **-0.60** | **+651** | **-0.53** | **+465** |

As shown in Table 5 and Figures 3 and 4, there is a measurable buffering effect associated with the HMB addition to PediaSure® powder; that is, the pH drop is not as great in the sample including HMB and the corresponding [H+] increase is not as great.

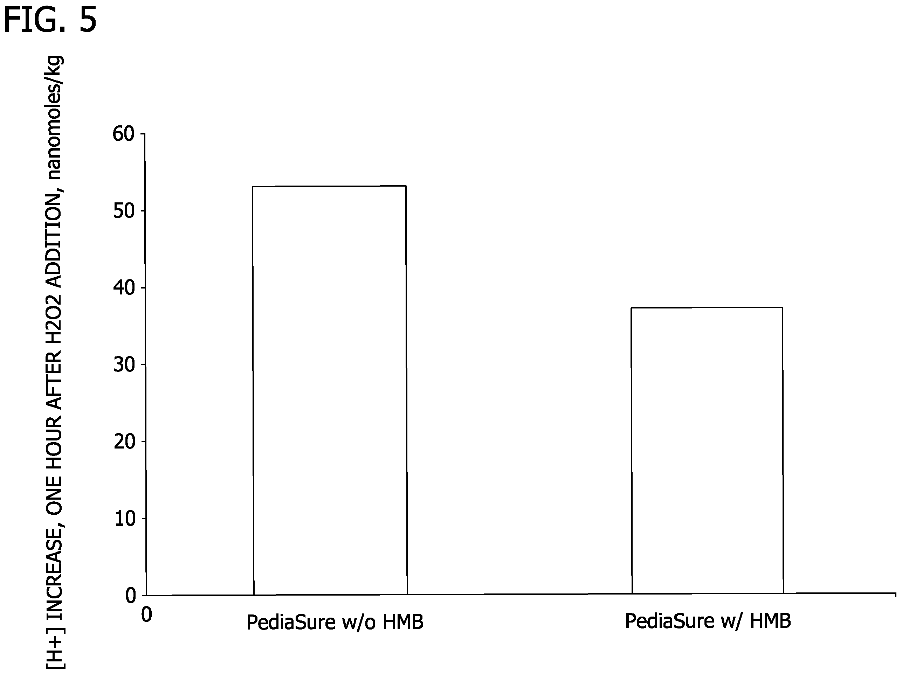

In a second experiment, two additional samples of reconstituted PediaSure® powder are prepared. The first sample is a control sample of reconstituted PediaSure® nutritional powder and the second sample is the powder fortified with HMB as described above. In this second experiment, 1.32 mg of hydrogen peroxide (H2O2) per kg of reconstituted powder is added and the pH is measured after one hour at room temperature (RT) Again, the [H+] concentrations are calculated from the pH values. The results are shown in Table 6 and in Figure 5.

**Table 6**

| **Time after H2O2 addition (at 1.32 mg/kg)** | **PediaSure® Powder w/o HMB addition** | | **PediaSure® Powder w/ HMB addition** | |
|---|---|---|---|---|
| | pH | [H+], nmoles/kg | pH | [H+], nmoles/kg |
| 0-time | 6.64 | 228 | 6.68 | 208 |
| 1 hour/RT | 6.55 | 281 | 6.61 | 245 |
| **Change** | **-0.09** | **+53** | **-0.07** | **+37** |

As shown in Table 6 and Figure 5, the addition of HMB to PediaSure® powder exerts a measureable resistance to the pH lowering that generally accompanies peroxide-initiated oxidation in reconstituted PediaSure® powder and other nutritional products.

Additionally, as noted above, the resistance to pH lowering decreases the release of bound minerals (e.g., phosphate-bound calcium), which, in ionic form (i.e., if released from the phosphate salt), could compromise physical stability of the powder and increase catalytic oxidation of metal components of the reconstituted powders such as iron and copper. Additionally, this resistance to pH lowering could inhibit protein precipitation from the reconstituted nutritional powder and inhibit vitamin C destabilization, further leading to product instability.

### Example 5

In this Example, the buffering capacity of adding HMB to the commercially available Ensure® nutritional emulsion in dialyzates is analyzed.

HMB is obtained as described in Example 1. The Ensure® emulsions (available from Abbott Nutrition, Columbus, Ohio) are fortified with either citrate and/or HMB. The dialyzates are prepared by: cutting a 30-cm length of 6000-8000 MWCO dialysis tubing (available as #D-1614-4 from Baxter) and rinsing the tubing thoroughly with Milli-Q Plus water. One end of the tubing is tied and 50 mL of Ensure® with HMB is added. The opposing end of the tubing is then tied and the tubing is placed in a 1-L bottle containing 950 mL of Milli-Q Plus water. The tubing is stirred continuously for approximately 15 hours at room temperature (RT).

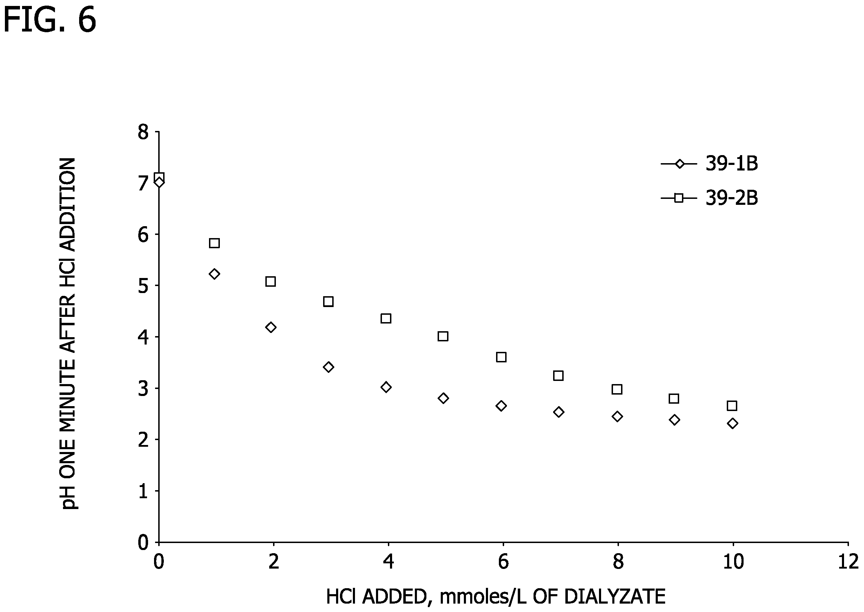

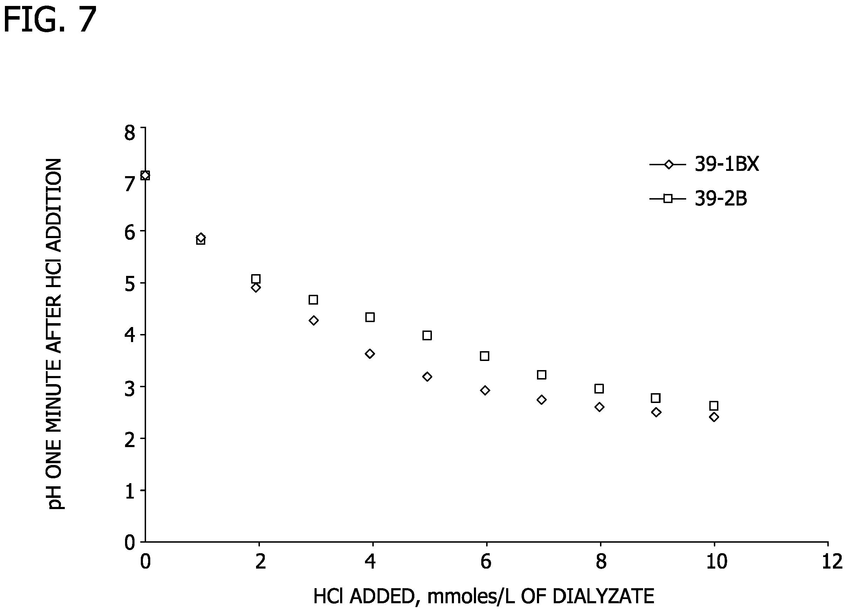

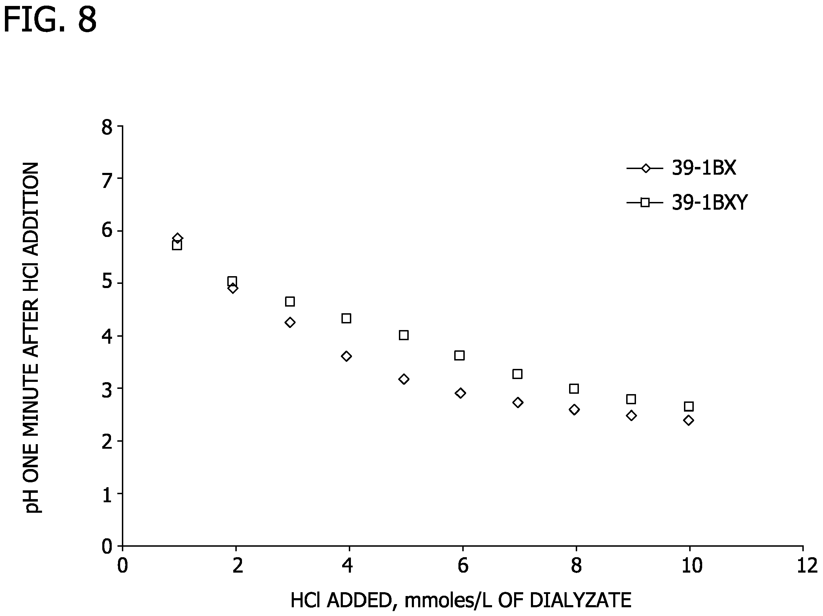

The liquid outside of the tubing (i.e., dialyzate) is then analyzed for HMB and citrate presence using organic acid HPLC. Additionally, the buffering capacity is determined by measuring pH versus HCl titration. The results are shown in Table 7, as well as in Figures 6-8.

**Table 7**

| **Sample Emulsion** | **HMB, mg per liter of dialyzate** | **Citrate, mg per liter of dialyzate** | **pH drop after addition of 5 mM HCl** |
|---|---|---|---|
| 39-2B | 263 | 264 | 3.10 units |
| 39-1B (control) | 0 | 148 | 4.23 units |
| 39-1BX* | 0 | 264 | 3.91 units |
| 39-1BXY** | 263 | 264 | 3.07 units |

| | | | |
|---|---|---|---|
| *39-1B dialyzate to which citrate is added to a concentration of 264 mg/L **39-1BX to which HMB is added to a concentration of 263 mg/L | | | |

As shown in Table 7 and Figures 6-8, the presence of HMB in the 39-2B dialyzate significantly increases its buffering capacity in comparison to the 39-1B dialyzate. The presence of HMB in the dialyzate demonstrates that the HMB is soluble (as opposed to simply being suspended, or being bound in micelles) and thereby available for reaction with soluble iron to form the insoluble ferric HMB thus reducing the concentration of soluble iron.

The concentration of calcium is also analyzed in the dialyzate. As shown in Table 8, the 39-2B dialyzate calcium concentration is greater than 2 times that of the 39-1B dialyzate calcium concentration, suggesting that calcium availability may be significantly greater in the emulsion with HMB addition (39-2B) as compared to the control (39- IB).

**Table 8**

| Sample Emulsion | Calcium, mg per liter of dialyzate | Dialyzate calcium, mg per kg of emulsion |
|---|---|---|
| 39-1B (w/o HMB) | 8.23 | 141 |
| 39-2b (W/ HMB) | 17.0 | 291 |

### Examples 6-10

Examples 6-10 illustrate nutritional powders not according to the invention including calcium HMB, the ingredients of which are listed in the table below. These products are prepared by spray drying methods in separate batches, are reconstituted with water prior to use to the desired target ingredient concentrations. All ingredient amounts are listed as kg per 1000 kg batch of product, unless otherwise specified.

| **Ingredient** | **Ex.6** | **Ex.7** | **Ex.8** | **Ex.9** | **Ex.10** |
|---|---|---|---|---|---|
| Maltodextrin (D.E. 19-24) | 436.7 | 436.7 | 436.7 | 436.7 | 436.7 |
| Sucrose | 145.5 | 145.5 | 145.5 | 145.5 | 145.5 |
| Calcium Caseinate | 129.1 | 129.1 | 129.1 | 129.1 | 129.1 |
| Isolated Soy Protein | 57.7 | 57.7 | 57.7 | 61.7 | 57.7 |
| FOS Powder | 33.6 | 33.6 | 33.6 | 33.6 | 32.6 |
| HO sunflower oil | 59.9 | 55.5 | 61.24 | 57.2 | 62.58 |
| Calcium HMB.H₂O | 31.6 | 34.6 | 28.6 | 27.6 | 32.6 |
| Canola Oil | 55.1 | 53.7 | 56.4 | 52.42 | 57.78 |
| Soy Oil | 26.7 | 26.0 | 27.37 | 25.36 | 28.04 |
| Potassium Citrate | 10.3 | 10.3 | 10.3 | 10.3 | 10.3 |
| Sodium Citrate | 5.8 | 5.8 | 5.8 | 5.8 | 5.8 |
| Potassium Chloride | 5.2 | 5.2 | 5.2 | 5.2 | 5.2 |
| Magnesium Chloride | 4.7 | 4.7 | 4.7 | 4.7 | 4.7 |
| Potassium hydroxide | 3.1 | 3.1 | 3.1 | 3.1 | 3.1 |
| Sodium phosphate dibasic dihydrate | 3.0 | 3.0 | 3.0 | 3.0 | 3.0 |
| Sodium chloride | 2.5 | 2.5 | 2.5 | 2.5 | 2.5 |
| Choline Chloride | 1.8 | 1.8 | 1.8 | 1.8 | 1.8 |
| Vanilla Flavor | 1.8 | 1.8 | 1.8 | 1.8 | 1.8 |
| Sodium phosphate monobasic monohydrate | 1.6 | 1.6 | 1.6 | 1.6 | 1.6 |
| Potassium phosphate dibasic trihydrate | 1.1 | 1.1 | 1.1 | 1.1 | 1.1 |
| FlavoR | 1.0 | 1.0 | 1.0 | 1.0 | 1.0 |
| Vitamin premix | 1.0 | 1.0 | 1.0 | 1.0 | 1.0 |
| Ascorbyl palmitate | 0.243 | 0.243 | 0.243 | 0.243 | 0.243 |
| Ascorbic acid | 0.240 | 0.240 | 0.240 | 0.240 | 0.240 |
| Tocopherol-2 antioxidant | 0.116 | 0.116 | 0.116 | 0.116 | 0.116 |
| Ferrous sulfate | 0.010 | 0.090 | 0.030 | 0.020 | 0.070 |
| Vitamin premix | 0.065 | 0.065 | 0.065 | 0.065 | 0.065 |
| Zinc sulfate monohydrate | 0.057 | 0.057 | 0.057 | 0.057 | 0.057 |
| Manganese sulfate | 0.045 | 0.045 | 0.045 | 0.045 | 0.045 |
| Mineral mix copper sulfate | 0.035 | 0.035 | 0.035 | 0.035 | 0.035 |
| Beta carotene 30% | 0.005 | 0.005 | 0.005 | 0.005 | 0.005 |
| Chromium chloride | 0.001 | 0.001 | 0.001 | 0.001 | 0.001 |
| Sodium molybdate | 0.0012 | 0.0012 | 0.0012 | 0.0012 | 0.0012 |
| Potassium iodide | 0.001 | 0.001 | 0.001 | 0.001 | 0.001 |
| Sodium selenite | 0.0004 | 0.0004 | 0.0004 | 0.0004 | 0.0004 |
| Citric acid | AN | AN | AN | AN | AN |
| Potassium hydroxide | AN | AN | AN | AN | AN |
| Magnesium sulfate dry | AN | AN | AN | AN | AN |
| Ultra micronized tricalcium phosphate | AN | AN | AN | AN | AN |
| Ascorbic acid | AN | AN | AN | AN | AN |

| | | | | | |
|---|---|---|---|---|---|
| AN = As Needed | | | | | |

### Examples 11-15

Examples 11-15 illustrate nutritional emulsion embodiments not according to the invention, the ingredients of which are listed in the table below. All amounts are listed as kilogram per 1000 kilogram batch of product, unless otherwise specified.

| **Ingredient** | **Ex.11** | **Ex.12** | **Ex.13** | **Ex.14** | **Ex.15** |
|---|---|---|---|---|---|
| Water | Q.S | Q.S | Q.S | Q.S | Q.S |
| Sucrose | 89.3 | 89.3 | 89.3 | 89.3 | 89.3 |
| Maltodextrin | 29.7 | 29.7 | 29.7 | 29.7 | 29.7 |
| Sodium Caseinate | 25.9 | 25.9 | 25.9 | 25.9 | 25.9 |
| Milk Protein Caseinate | 19.1 | 19.1 | 19.1 | 19.1 | 19.1 |
| Soy Protein Isolate | 11.9 | 11.9 | 9.9 | 12.9 | 13.9 |
| Potassium Citrate | 7.9 | 7.9 | 7.9 | 7.9 | 7.9 |
| Soy Oil | 11.1 | 9.9 | 11.4 | 10.7 | 11.6 |
| Calcium HMB.H₂0 | 6.7 | 7.7 | 8.7 | 5.7 | 4.7 |
| Canola Oil | 10.2 | 10.0 | 10.5 | 9.8 | 10.7 |
| Corn Oil | 9.3 | 9.1 | 9.6 | 8.9 | 9.8 |
| Whey Protein Concentrate | 3.5 | 3.5 | 3.5 | 3.5 | 3.5 |
| Magnesium Phosphate Dibasic | 3.1 | 3.1 | 3.1 | 3.1 | 3.1 |
| Flavoring Agent | 2.0 | 2.0 | 2.0 | 2.0 | 2.0 |
| Stabilizer | 2.0 | 2.0 | 2.0 | 2.0 | 2.0 |
| Soy Lecithin | 1.5 | 1.5 | 1.5 | 1.5 | 1.5 |
| Sodium Phosphate Dibasic Dihydrate | 1.3 | 1.3 | 1.3 | 1.3 | 1.3 |
| Potassium Phosphate Dibasic | 0.985 | 0.985 | 0.985 | 0.985 | 0.985 |
| Potassium Chloride | 0.729 | 0.729 | 0.729 | 0.729 | 0.729 |
| Choline Chloride | 0.480 | 0.480 | 0.480 | 0.480 | 0.480 |
| Ascorbic Acid | 0.469 | 0.469 | 0.469 | 0.469 | 0.469 |
| Calcium Carbonate | 0.451 | 0.451 | 0.451 | 0.451 | 0.451 |
| Caramel Flavor | 0.450 | 0.450 | 0.450 | 0.450 | 0.450 |
| N&A Dairy Cream 32122 | 0.450 | 0.450 | 0.450 | 0.450 | 0.450 |
| UTM/TM Premix | 0.367 | 0.367 | 0.367 | 0.367 | 0.367 |
| 45% Potassium Hydroxide | 0.323 | 0.323 | 0.323 | 0.323 | 0.323 |
| Carrageenan | 0.200 | 0.200 | 0.200 | 0.200 | 0.200 |
| Water Soluble Vitamin Premix | 0.185 | 0.185 | 0.185 | 0.185 | 0.185 |
| Vitamin DEK Premix | 0.067 | 0.067 | 0.067 | 0.067 | 0.067 |
| Sodium Chloride | 0.060 | 0.060 | 0.060 | 0.060 | 0.060 |
| Gellan Gum | 0.050 | 0.050 | 0.050 | 0.050 | 0.050 |
| Vitamin A Palmitate in corn oil | 0.0082 | 0.0082 | 0.0082 | 0.0082 | 0.0082 |
| Corn oil carrier | Q.S. | Q.S. | Q.S. | Q.S. | Q.S. |
| Vitamin D₃ in corn oil | 19 mg | 19 mg | 19 mg | 19 mg | 19 mg |
| Potassium Iodide | 194 mg | 194 mg | 194 mg | 194 mg | 194 mg |

## Claims

1. Use of a nutritional powder to provide a reconstituted nutritional liquid by mixing the nutritional powder with water, the nutritional powder comprising from 0.1% to 10% by weight beta-hydroxy-beta- methylbutyrate, from 0.001 % to 5% by weight iron, and from 5.0% to 10% by weight fat, wherein the fat comprises from 0.01% to 5% by weight of a long chain polyunsaturated fatty acid derived from fish oil, and wherein the weight ratio of beta-hydroxy-beta-methylbutyrate: iron is from 150:1 to 600:1.

2. Use according to claim 1, the nutritional powder further comprising at least one of vitamins and minerals.

3. Use according to claim 2, wherein the nutritional powder comprises at least one vitamin selected from the group consisting of folic acid, vitamin B12, vitamin C, vitamin D, vitamin E, and combinations thereof.

4. Use according to claim 3 wherein the nutritional powder comprises from 0.0000001% to 0.00001% by weight of vitamin D.

5. Use according to claim 3 wherein the nutritional powder comprises from 0.000001% to 0.001% by weight of folic acid.

6. Use according to any of claims 1 to 5 wherein the nutritional powder comprises casein.

7. Use according to any of claims 1 to 6 wherein the nutritional powder comprises a weight ratio of beta-hydroxy- beta-methylbutyrate: iron of from 200:1 to 600:1.

8. Use according to any of claims 1 to 7, wherein the nutritional powder is packaged in a metal container wherein the metal container comprises at least one metal component selected from iron, copper, zinc and manganese, which can migrate from the metal container into the nutritional powder over time, and wherein nutritional powder is in at least partial contact with the metal.

## Patentansprüche

1. Verwendung eines Nahrungsmittelpulvers zum Bereitstellen einer rekonstituierten Nahrungsmittelflüssigkeit durch Mischen des Nahrungsmittelpulvers mit Wasser, wobei das Nahrungsmittelpulver 0,1 bis 10 Gew.-% beta-Hydroxy-beta-methylbutyrat, 0,001 bis 5 Gew.-% Eisen und 5,0 bis 10 Gew.-% Fett umfasst, wobei das Fett 0,01 bis 5 Gew.-% von einer langkettigen, mehrfach ungesättigten Fettsäure, die aus Fischöl gewonnen wird, umfasst und wobei das Gewichtsverhältnis von beta-Hydroxy-beta-methylbutyrat:Eisen 150:1 bis 600:1 beträgt.

2. Verwendung nach Anspruch 1, wobei das Nahrungsmittelpulver weiter zumindest eines von Vitaminen und Mineralien umfasst.

3. Verwendung nach Anspruch 2, wobei das Nahrungsmittelpulver zumindest ein Vitamin ausgewählt aus der Gruppe, bestehend aus Folsäure, Vitamin B12, Vitamin C, Vitamin D, Vitamin E und Kombinationen davon, umfasst.

4. Verwendung nach Anspruch 3, wobei das Nahrungsmittelpulver 0,0000001 bis 0,00001 Gew.-% Vitamin D umfasst.

5. Verwendung nach Anspruch 3, wobei das Nahrungsmittelpulver 0,000001 bis 0,001 Gew.-% Folsäure umfasst.

6. Verwendung nach einem der Ansprüche 1 bis 5, wobei das Nahrungsmittelpulver Kasein umfasst.

7. Verwendung nach einem der Ansprüche 1 bis 6, wobei das Nahrungsmittelpulver ein Gewichtsverhältnis von beta-Hydroxy-beta-methylbutyrat:Eisen von 200:1 bis 600:1 umfasst.

8. Verwendung nach einem der Ansprüche 1 bis 7, wobei das Nahrungsmittelpulver in einem Metallbehälter verpackt ist, wobei der Metallbehälter zumindest eine Metallkomponente ausgewählt aus Eisen, Kupfer, Zink und Mangan umfasst, die mit der Zeit von dem Metallbehälter in das Nahrungsmittelpulver wandern kann, und wobei Nahrungsmittelpulver in zumindest teilweisem Kontakt mit dem Metall steht.

## Revendications

1. Utilisation d'une poudre nutritionnelle pour fournir un liquide nutritionnel reconstitué en mélangeant la poudre nutritionnelle avec de l'eau, la poudre nutritionnelle comprenant 0,1% à 10% en poids de bêta-hydroxy-bêta-méthylbutyrate, 0,001% à 5% en poids de fer, et 5,0% à 10% en poids de matière grasse, où la matière grasse comprend 0,01% à 5% en poids d'un acide gras polyinsaturé à longue chaîne dérivé de l'huile de poisson, et où le rapport pondéral bêta-hydroxy-bêta-méthylbutyrate : fer est compris entre 150 : 1 et 600 : 1.

2. Utilisation selon la revendication 1, la poudre nutritionnelle comprenant en outre des vitamines et/ou des minéraux.

3. Utilisation selon la revendication 2, dans laquelle la poudre nutritionnelle comprend au moins une vitamine choisie dans le groupe consistant en l'acide folique, la vitamine B12, la vitamine C, la vitamine D, la vitamine E et des combinaisons de ceux-ci.

4. Utilisation selon la revendication 3, dans laquelle la poudre nutritionnelle comprend 0,0000001% à 0,00001% en poids de vitamine D.

5. Utilisation selon la revendication 3, dans laquelle la poudre nutritionnelle comprend 0,000001% à 0,001% en poids d'acide folique.

6. Utilisation selon l'une des revendications 1 à 5 dans laquelle la poudre nutritionnelle comprend de la caséine.

7. Utilisation selon l'une des revendications 1 à 6 dans laquelle la poudre nutritionnelle comprend un rapport pondéral bêta-hydroxy-bêta-méthylbutyrate : fer compris entre 200 : 1 et 600 : 1.

8. Utilisation selon l'une des revendications 1 à 7, dans laquelle la poudre nutritionnelle est emballée dans un récipient métallique où le récipient métallique comprend au moins un composant métallique choisi entre le fer, le cuivre, le zinc et le manganèse, qui peuvent migrer du récipient métallique dans la poudre nutritionnelle au fil du temps, et dans laquelle la poudre nutritionnelle est en contact au moins partiel avec le métal.
